# EUROPEAN PATENT SPECIFICATION

(11) **EP 0 437 595 B1**
(45) Date of publication and mention of the grant of the patent: **27.07.1994**
(21) Application number: 90912879.5
(22) Date of filing: 28.07.1990
(51) Int. Cl.: C09K 19/30

(54) **FLUORO-CHLORO-BENZENE DERIVATIVES**
FLUOR-CHLOR-BENZOL-DERIVATE
DERIVES DE FLUORO-CHLORO-BENZENE

(30) Priority: 10.08.1989 GB 8918275
(43) Date of publication of application: 24.07.1991
(73) Proprietor: MERCK PATENT GmbH, D-64271 Darmstadt (DE)
(72) Inventor: GREENFIELD, Simon, Poole BH17 7YA (GB); COATES, David, Dorset BH21 3SW (GB)
(86) International application number: EP9001234
(87) International publication number: WO9102043

(56) References cited:
- GB-A- 2 071 131
- PATENT ABSTRACTS OF JAPAN, vol. 8, no. 251 (-252) (1688), 16. November 1984; & JP-A-59 128 354

## Description

The invention relates to fluoro-chloro-benzene derivatives of the formula I 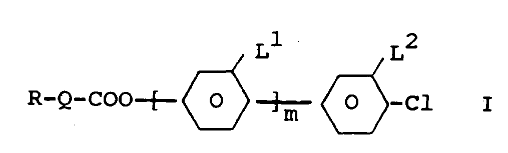
wherein
- R: denotes an alkyl residue of up to 12 C atoms wherein one or two non-adjacent CH₂ groups may also be replaced by -O- and/or -HC=CH-,
- Q: is 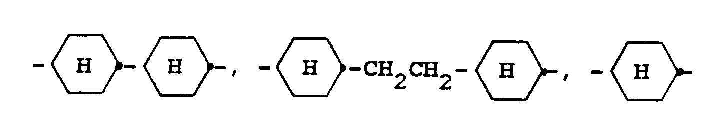 or a single bond,
- m: is 0 or 1, and
one of the radicals L¹ and L² present in the molecule is F and the other radical - if any - is hydrogen,
and also to liquid crystalline media being a mixture of at least 2 compounds, characterized in that at least one compound is a fluorinated benzene derivative according to formula I.

The invention was based on the object of discovering new stable liquid crystal or mesogenic compounds which are suitable as components of liquid crystalline media and, in particular, have advantageous values for optical and dielectric anisotropy combined with low viscosity and high nematogenity.

Similar chloro-benzene derivatives without lateral fluorine substitution are known: 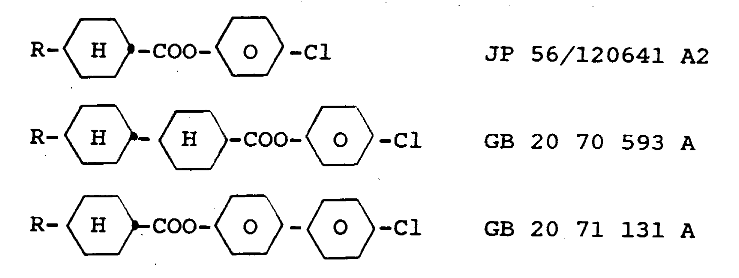
The following laterally fluorinated derivatives bearing the fluorine in m-position are also known: 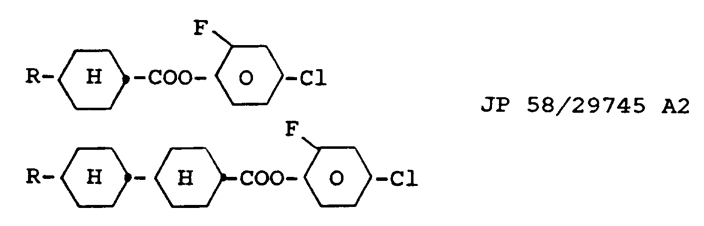
It has now been found that laterally fluorinated compounds of formula I are highly suitable as polar components of liquid crystalline media. In particular, they have especially advantageous values of optical and dielectric anisotropy and are not strongly smectogenic. It is also possible to obtain stable liquid crystal phases with a broad nematic mesophase range including a good deep temperature behaviour, a high resistivity and a comparatively low viscosity with the aid of these compounds.

Depending on the choice of R, Q, m, L¹ and L², the compounds of the formula I can be used as the base materials from which liquid crystal media are predominantly composed; however, it is also possible for compounds of the formula I to be added to liquid crystal base materials of other classes of compounds, for example in order to influence the dielectric and/or optical anisotropy and/or the viscosity and/or the nematic mesophase range of such a dielectric.

The compounds of the formula I are colourless in the pure state and are liquid crystalline in a temperature range which is favourably placed for electrooptical use. They are very stable towards chemicals, heat and light.

The invention thus relates to the benzene derivatives of the formula I, to liquid crystalline media with at least two liquid crystalline components, wherein at least on component is a compound of the formula I and to liquid crystal display devices containing such media.

Above and below, R, Q, m, L¹ and L² have the meaning given unless expressly indicated otherwise. Cy is trans-1,4-cyclohexylene.

The compounds of the formula I include benzene derivatives of the formulae Ia to Id: 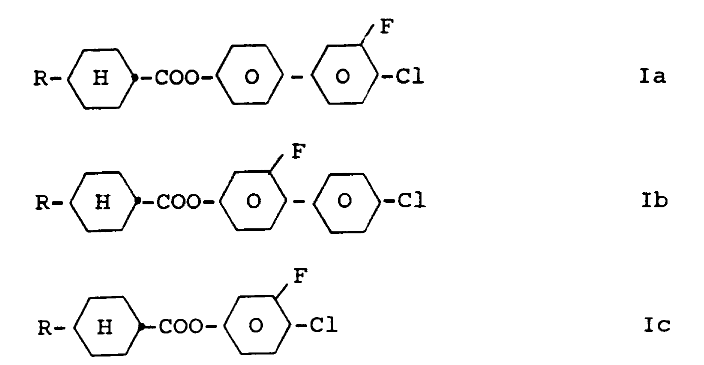 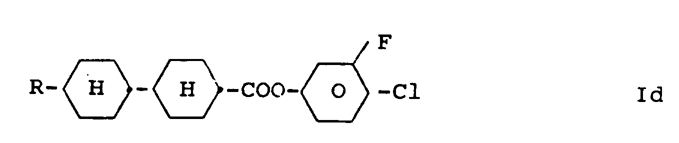
Also preferred are the following compounds: 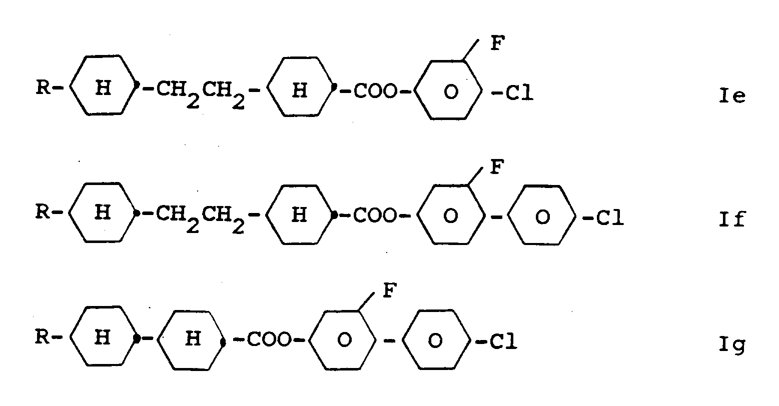
R is preferably alkyl, alkoxy, oxaalkyl or alkenyl and can exhibit a straight-chain or branched structure.

Alkyl or alkoxy preferably are straight-chain and have 2, 3, 4, 5, 6 or 7 C atoms. Accordingly they are preferably ethyl, propyl, butyl, pentyl, hexyl, heptyl, ethoxy, propoxy, butoxy, pentoxy, hexoxy or heptoxy, also methyl, octyl, nonyl, decyl, undecyl, dodecyl, tridecyl, tetradecyl, pentadecyl, methoxy, octoxy, nonoxy, decoxy, undecoxy, dodecoxy, tridecoxy or tetradecoxy.

Oxaalkyl is preferably straight-chain 2-oxapropyl (= methoxymethyl), 2-(= ethoxymethyl) or 3-oxybutyl (= 2-methoxyethyl), 2-, 3- or 4-oxapentyl, 2-, 3-, 4-or 5-oxahexyl, 2-, 3-, 4- 5- or 6-oxaheptyl, 2-, 3-, 4-, 5-, 6- or 7-oxaoctyl, 2-, 3-, 4-, 5-, 6-, 7- or 8-oxanonyl or 2-, 3-, 4-, 5-, 6-, 7-, 8-, or 9-oxadecyl.

Alkenyl is preferably straight-chain and has 2 or 10 C atoms. It is accordingly, in particular, vinyl, prop-1-or prop-2-enyl, but-1-, -2- or -3-enyl, pent-1-, -2-, -3-or -4-enyl, hex-1-, -2-, -3-, -4- or -5-enyl, hept-1-, -2-, -3-, -4-, -5- or -6-enyl, oct-1-, -2-, -3-, -4-, -5--6- or -7-enyl, non-1-, -2-, -3-, -4-, -5-, -6-, -7- or -8-enyl or dec-1-, -2-, -3-, -4-, -5-, -6-, -7-, -8- or -9-enyl.

Compounds of the formula I containing a branched terminal group can occasionally be of importance because of an improved solubility in the customary liquid crystal base materials, but in particular as chiral doping substances if they are optically active.

Branched groups of this type as a rule contain not more than one chain branching. Preferred branched radicals are isopropyl, 2-butyl (= 1-methylpropyl), isobutyl (= 2-methylpropyl, 2-methylbutyl, isopentyl, (= 3-methylbutyl), 2-methylpentyl, 2-ethylhexyl, 2-propylpentyl, 2-octyl, isopropoxy, 2-methylpropoxy, 2-methylbutoxy, 3-methylbutoxy, 2-methylpentoxy, 3-methylpentoxy, 2-ethylhexoxy, 2-methylhexoxy, 1-methylhexoxy, 1-methylheptoxy (= 2-octyloxy), 2-oxa-3-methylbutyl, 3-oxa-4-methylpentyl, 4-methylhexyl, 2-nonyl, 2-decyl, 2-dodecyl, 6-methyl-octoxy, oxy, 2-methyl-3-oxapentyl and 2-methyl-3-oxahexyl.

In the case of compounds with a branched terminal group R, formula I includes both the optical antipodes and racemates as well as mixtures thereof.

If Q denotes a single bond m is preferably 1. Especially preferred are compounds with m = 1, L¹ = F and L² = H.

Of the compounds of the formula I and subformulae thereof, those in which at least one of the radicals contained therein has one of the preferred meanings given are preferred.

The compounds of the formula I are prepared by methods which are known per se, such as are described in the literature (for example in the standard works, such as Houben-Weyl, Methoden der Organischen Chemie [Methods of Organic Chemistry], Georg Thieme Verlag, Stuttgart), and in particular under reaction conditions which are known and suitable for the reactions mentioned. Variants which are known per se and are not mentioned in more detail here can also be used in this connection.

If desired, the starting materials can also be formed in situ, such that they are not isolated from the reaction mixture but are immediately reacted further to give the compounds of the formula I.

The carboxylic acids of the formula

R-Q-COOH

and their reactive derivatives, e.g. the corresponding acyl chlorides, are well known in the art as well as the reaction conditions for esterification with fluorochloro phenols of formula 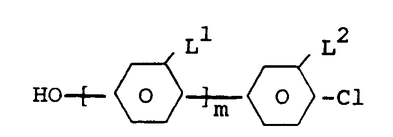
or their reactive derivatives.

A preferred route for preparation of the fluoro-chloro phenols is shod in the following schemes 1, 2 and 3: 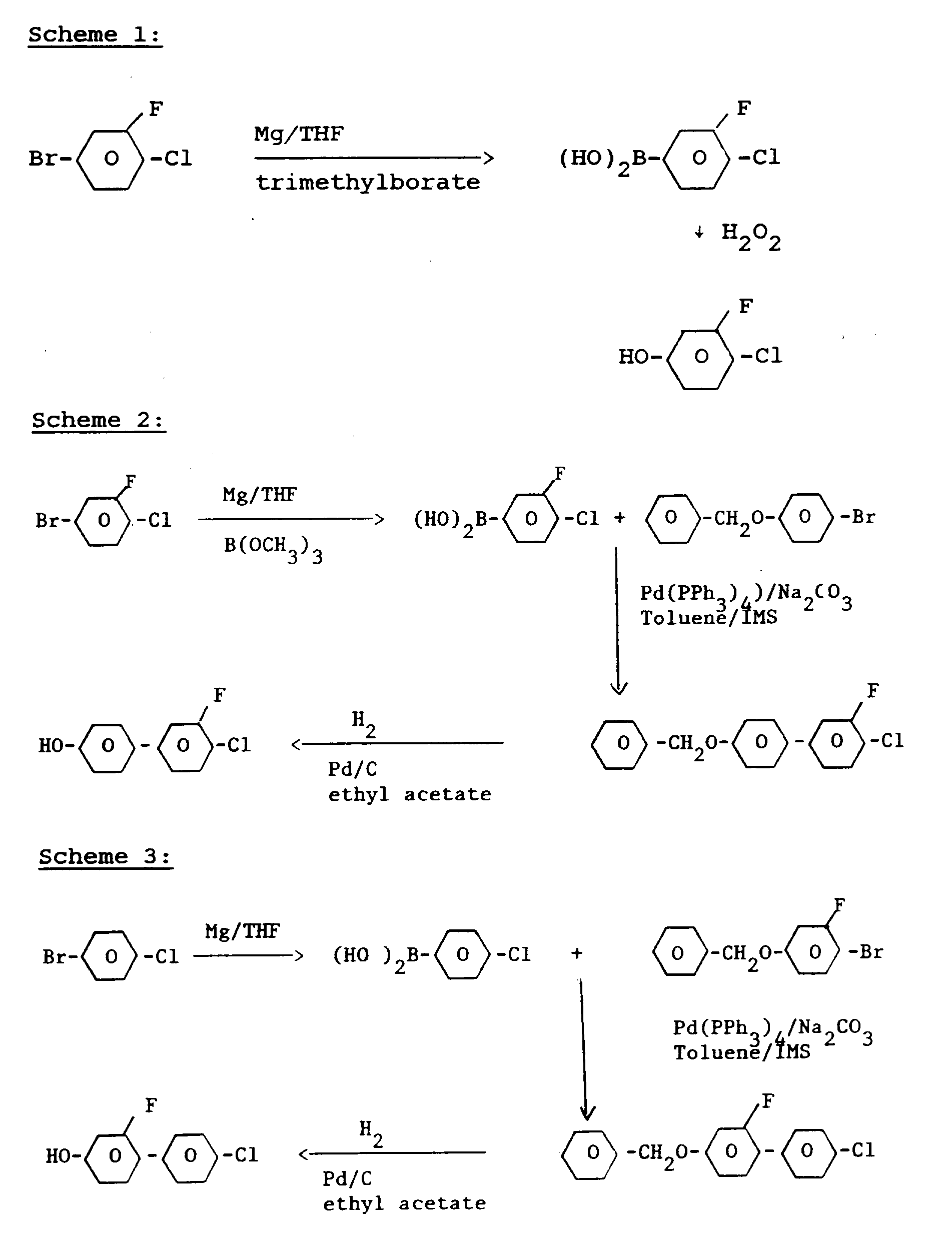
All starting materials are known or prepared in analogy to known starting materials.

Other routes are apparent to the skilled worker. All these steps and the corresponding reaction conditions are known to the skilled worker.

In addition to one or more compounds for formula I the liquid crystal media according to the invention preferably contain 2-40 components and in particular 4-30 components. Liquid crystal media being composed of one or more compounds of formula I and 7-25 other components are especially preferred.

These additional components are preferably chosen from the nematic or nematogenic (monotropic or isotropic) substances; in particular from the classes of azoxybenzenes, benzylideneanilines, biphenyls, terphenyls, phenyl or cyclohexyl benzoates, phenyl or cyclohexyl cyclohexane-carboxylates, phenyl or cyclohexyl cyclohexylbenzoates, phenyl or cyclohexyl cyclohexylcyclohexanecarboxylates, cyclohexylphenylbenzoates, cyclohexylphenyl cyclohexane-carboxylates, cyclohexylphenyl cyclohexylcyclohexanecarboxylates, phenylcyclohexanes, cyclohexylbiphenyls, phenylcyclohexylcyclohexanes, cyclohexylcyclohexanes, cyclohexylcyclohexenes, cyclohexylcyclohexylcyclohexene, 1,4-bis-cyclohexylbenzenes, 4,4'-bis-cyclohexylbiphenyls, phenyl- or cyclohexylpyrimidines, phenyl- or cyclohexyl-pyridines, phenyl- or cyclohexyldioxanes, phenyl- or cyclohexyl-1,3-dithianes, 1,2-diphenylethanes, 1,2-di-cyclohexylethanes, 1-phenyl-2-cyclohexylethanes, 1-cyclohexyl-2-(4-phenyl-cyclohexyl)-ethanes, 1-cyclohexyl-2-biphenylethanes, 1-phenyl-2-cyclohexyl-phenylethanes, optionally halogenated stilbenes, benzyl phenyl ethers, tolanes and substituted cinnamic acids.

The 1,4-phenylene groups of these compounds may be fluorinated.

The most important compounds which are possible constituents of liquid crystal media according to the invention can be characterized by the formalae 1, 2, 3, 4 and 5:

R'-L-U-R'' 1

R'-L-COO-U-R'' 2

R'-L-OOC-U-R'' 3

R'-L-CH₂CH₂-U-R'' 4

R'-L-C≡C-U-R'' 5

In the formulae 1, 2, 3, 4 and 5 L and U may be equal or different from each other. L and U independently from each other denote a bivalent residue selected from the group consisting of -Phe-, -Cyc-, -Phe-Phe-, -Phe-Cyc-, -Cyc-Cyc-, -Pyr-, -Dio-, -G-Phe-, -G-Cyc- and their mirror images; in this compilation of residues Phe denotes unsubstituted or fluorinated 1,4-phenylen, Cyc trans-1,4-cyclohexylene or 1,4-cyclohexenylen, Pyr pyrimidine-2,5-diyl or pyridine-2,5-diyl, Dio 1,3-dioxane-2,5-diyl and G 2-(trans-1,4-cyclohexyl)-ethyl, pyrimidine-2,5-diyl, pyridine-2,5-diyl or 1,3-dioxane-2,5-diyl.

One of the residues L and U is preferably Cyc, Phe or Pyr. U preferably denotes Cyc, Phe or Phe-Cyc. The liquid crystal media according to the invention preferably contain one or more components selected from the compounds of formulae 1, 2, 3, 4 and 5 with L and U meaning Cyc, Phe and Pyr, said liquid crystal media further containing at the same time one ore more components selected from the compounds of formulae 1, 2, 3, 4 and 5 with one of the residues L and U denoting Cyc, Phe and Pyr and the other residue being selected from the group consisting of -Phe-Phe-, -Phe-Cyc-, -Cyc-Cyc-, -G-Cyc-, said liquid crystal media containing in addition to this optionally one or more components selected from the compounds of formulae 1, 2, 3, 4 and 5 with L and U being selected from the group consisting of -Phe-Cyc-, -Cyc-Cyc-, -G-Phe- and -G-Cyc.

In a preferred subgroup of the compounds of formulae 1, 2, 3, 4 and 5 (subgroup 1) R' and R'' are independently from each other alkyl, alkenyl, alkoxy, alkenoxy with up to 8 carbon atoms. R' and R'' differ from one another in most of these compounds, one of the residues usually being alkyl or alkenyl. In another preferred subgroup of the compounds of formulae 1, 2, 3, 4 and 5 (subgroup 2) R'' denotes -CN, -CF₃, -F, -Cl or -NCS while R' has the meaning indicated in subgroup 1 and is preferably alkyl or alkenyl. Other variants of the envisaged substituents in the compounds of formulae 1, 2, 3, 4 and 5 are also customary. Many such substances are commercially available. All these substances are obtainable by methods which are known from the literature or by analogous methods.

The liquid crystal media according to the invention preferably contain in addition to components selected from subgroup 1 also components of subgroup 2, the percentage of these components being as follows:
- subgroup 1:: 20 to 90 %, in particular 30 to 90 %
- subgroup 2:: 10 to 50 %, in particular 10 to 50 %
In these liquid crystal media the percentages of the compounds according to the invention and the compounds of subgroup 1 and 2 add up to give 100 %.

The media according to the invention preferably contain 1 to 40 %, in particular 5 to 30 % of the compounds according to the invention. Media containing more than 40 %, in particular 45 to 90 % of the compounds according to the invention are further preferred. The media contain preferably 3, 4 or 5 compounds according to the invention.

The media according to the invention are prepared in a manner which is customary per se. As a rule, the components are dissolved in one another, advantageously at elevated temperature. The liquid crystal media according to the invention can be modified by suitable additives so that they can be used in all the types of liquid crystal display devices. Such additives are known to the expert and are described in detail in the literature (H. Kelker/R. Hatz, Handbook of Liquid Crystals, Verlag Chemie, Weinheim, 1980). For example, it is possible to add pleochroic dyestuffs to prepare colored guest-host systems or substances for modifying the dielectric anisotropy, the viscosity and/or the orientation of the nematic phases.

The following examples are to be construed as merely illustrative and not limitative. m.p. = melting point, c.p. = clearing point. In the foregoing and in the following all parts and percentages are by weight and the temperatures are set forth in degrees Celsius. "Customary work-up" means that water is added, the mixture is extracted with methylene chloride, the organic phase is seperated off, dried and evaporated, and the product is purified by crystallization and/or chromatography.

Further are:
C: crystalline-solid state, S: smectic phase (the index denoting the typ of smectic phase), N: nematic phase, Ch: cholesteric phase, I: isotropic phase. The number being embraced by 2 of these symbols denotes the temperature of phase change.

### Example 1:

### Step 1

Magnesium (6.4 g, 0.263 moles) and THF (150 ml) were stirred under nitrogen. 1-bromo-3-fluoro-4-chlorobenzene (50 g, 0.239 moles) was added dropwise to the reaction mixture which was left to stir and reflux for 2 hrs. The reaction mixture was cooled to 0 °C, and trimethyl borate (30 ml, 0.263 moles) in THF (50 ml) was added keeping the temperature below 20 °C. The reaction mixture was left to stir in the ice bath for 30 mins, then quenched with acid solution (45 ml cHCl, 80 ml H₂O). The reaction mixture was extracted into ether, washed well with H₂O, then brine and finally the ether was evaporated to afford the crude boronic acid. The boronic acid was dissolved in ether (165 ml) and 30 % hydrogen peroxide solution (30 ml, 0.263 moles) was added dropwise over 30 minutes. This was then left to stir at room temperature for 16 hrs. The reaction mixture was extracted with ether, washed well with H₂O then with ferrous sulphate to remove excess H₂O₂. The organic layer was extracted with 1 M NaOH solution and the aqueous phase acidified with cH₂SO₄. The acidic solution was re-extracted into ether, dried and run down. The crude product was distilled to afford the phenol, bp 64°/2.5 mbar.

### Step 2

The phenol (1.84 g, 0.013 moles) and trans-4-(trans-4-n-propylcyclohexyl)-cyclohexane carboxylic acid (3.6 g, 0.0143 moles) were dissolved in DCM (30 ml) with trifluoroacetic anhydride (2 ml, 0.0143 moles). This was left to stand for 16 hrs. The reaction mixture was added to H₂O (30 ml), the organic layer was washed with NaHCO₃ solution, then with H₂O and dried and run down. After customary work-up 3-fluoro-4-chlorophenyl trans-4-(trans-4-n-propylcyclohexyl)-cyclohexane carboxylate is obtained, C 66.4 N 188.3 I.

### Examples 2 to 13:

In place of the used carboxylic acid and the fluoro-chloro-phenol other starting materials can be used for preparation of the following compounds in analogy to example 1:

| | R | Q | m | L¹ | L² |
|---|---|---|---|---|---|
| (2) | n-butyl | Cy-Cy | 0 | - | F |
| (3) | n-pentyl | Cy-Cy | 0 | - | F, K 63.4 N 186 I |
| (4) | n-heptyl | Cy-Cy | 0 | - | F |
| (5) | n-propyl | Cy | 0 | - | F, K 50.0 I |
| (6) | n-pentyl | Cy | 0 | - | F, K 50.4 I |
| (7) | n-heptyl | Cy | 0 | - | F |
| (8) | ethyl | Cy | 1 | H | F |
| (9) | n-propyl | Cy | 1 | H | F, K 72.4 S_{A} 133 N 182 I |
| (10) | n-pentyl | Cy | 1 | H | F |
| (11) | ethyl | Cy | 1 | F | H |
| (12) | n-propyl | Cy | 1 | F | H, K 77 N 177.4 I |
| (13) | n-pentyl | Cy | 1 | F | H |
| (14) | ethyl | Cy-Cy | 0 | - | F |
| (15) | n-pentyl | '-' | 1 | F | H K 23.7 I |
| (16) | n-propyl | '-' | 1 | F | H |
| (17) | ethyl | Cy-Cy | 1 | F | H |
| (18) | n-propyl | Cy-Cy | 1 | F | H |
| (19) | n-butyl | Cy-Cy | 1 | F | H |
| (20) | n-pentyl | Cy-Cy | 1 | F | H |
| (21) | n-propyl | Cy-C₂H₄-Cy | 1 | F | H |
| (22) | n-pentyl | Cy-C₂H₄-Cy | 1 | F | H |
| (23) | n-propyl | Cy-C₂H₄-Cy | O | - | F |
| (24) | n-pentyl | Cy-C₂H₄-Cy | 0 | - | F |

## Claims

1. Fluoro-chloro-benzene derivatives of the formula I 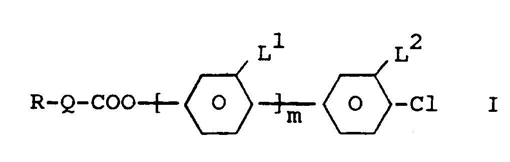 wherein
R denotes an alkyl residue of up to 12 C atoms wherein one or two non-adjacent CH₂ groups may also be replaced by -O- and/or -HC=CH-,
Q is 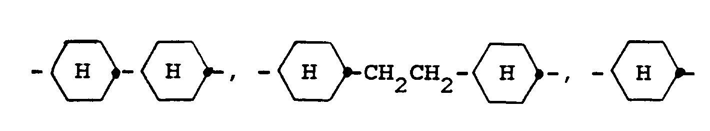 or a single bond,
m is 0 or 1, and
one of the radicals L¹ and L² present in the molecule is F and the other radical - if any - is hydrogen.

2. Benzene derivatives of claim 1, characterized by formula Ia 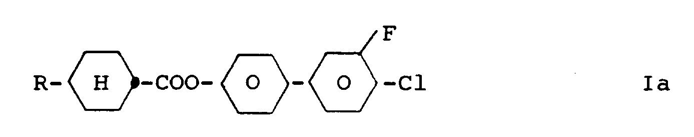 wherein R has the meaning given in claim 1.

3. Benzene derivatives of claim 1, characterized by formula Ib 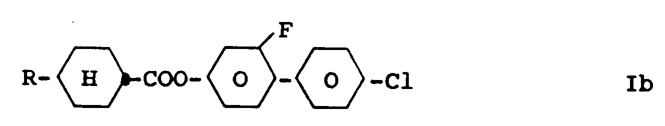 wherein R has the meaning given in claim 1.

4. Benzene derivatives of claim 1, characterized by formula Ic 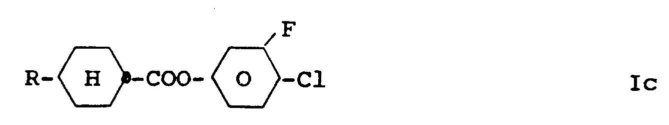 wherein R has the meaning given in claim 1.

5. Benzene derivatives of claim 1, characterized by formula Id 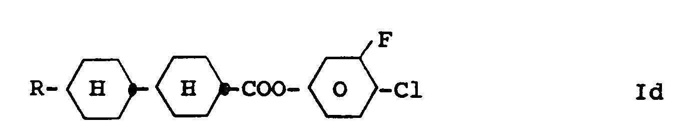 wherein R has the meaning given in claim 1.

6. Liquid crystalline medium being a mixture of at least two compounds, characterized in that at least one compound is a benzene derivative according to claim 1.

7. Liquid crystal display device, characterized in that it contains a liquid crystalline medium according to claim 6.

8. Electrooptical display device, characterized in that it contains a liquid crystalline medium according to claim 6.

## Patentansprüche

1. Fluor-chlor-Benzolderivate der Formel I 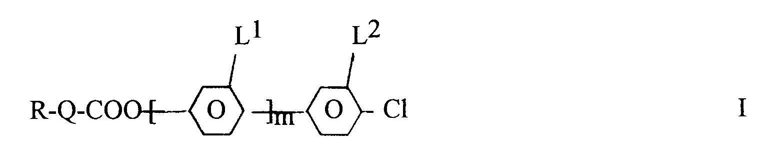 worin
R einen Alkylrest mit bis zu 12 C-Atomen, worin eine oder zwei nicht benachbarte CH₂-Gruppen durch -O- und/oder -CH=CH-ersetzt sein können,
Q 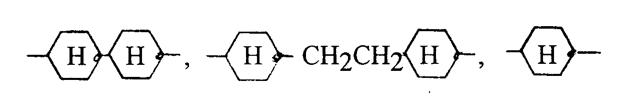 oder eine Einfachbindung,
m 0 oder 1 bedeutet, und
einer der im Molekül vorhandenen Reste L¹ und L²F und der andere Rest- falls vorhanden - Wasserstoff bedeutet.

2. Benzolderivate nach Anspruch 1, gekennzeichnet durch die Formel Ia 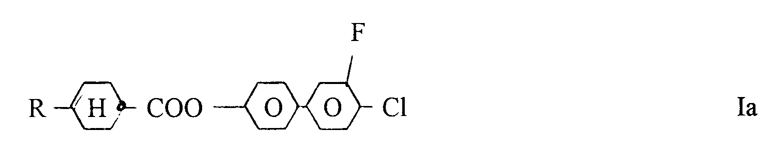 worin R die in Anspruch 1 angegebene Bedeutung hat.

3. Benzolderivate nach Anspruch 1, gekennzeichnet durch die Formel Ib 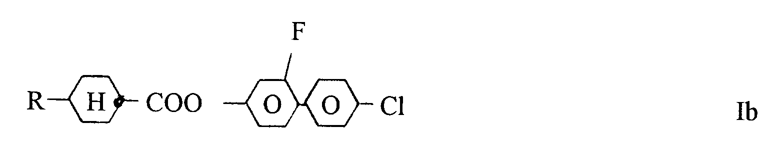 worin R die in Anspruch 1 angegebene Bedeutung hat.

4. Benzolderivate nach Anspruch 1, gekennzeichnet durch die Formel Ic 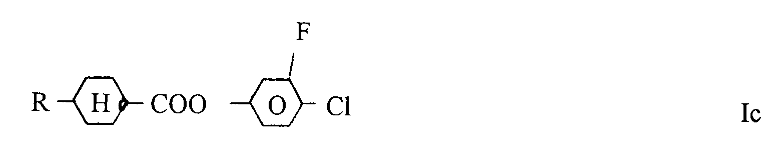 worin R die in Anspruch 1 angegebene Bedeutung hat.

5. Benzolderivate nach Anspruch 1, gekennzeichnet durch die Formel Id 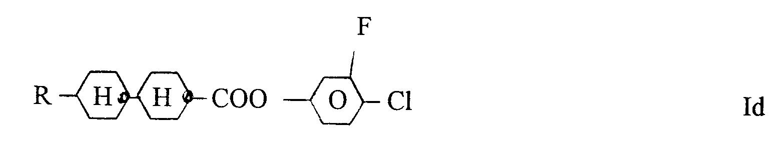 worin R die in Anspruch 1 angegebene Bedeutung hat.

6. Flüssigkristallines Medium aus einem Gemisch von mindestens zwei Verbindungen, dadurch gekennzeichnet, daß mindestens eine Verbindung ein Benzolderivat nach Anspruch 1 ist.

7. Flüssigkristallanzeige, dadurch gekennzeichnet, daß sie ein flüssigkristallines Medium nach Anspruch 6 enthält.

8. Elektrooptisches Anzeigeelement, dadurch gekennzeichnet, daß es ein flüssigkristallines Medium nach Anspruch 6 enthält.

## Revendications

1. Dérivés de fluoro-chloro-benzène de formule I 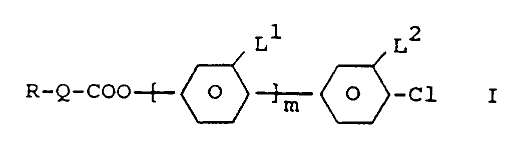 dans laquelle
R dénote un résidu alkyle ayant jusqu'à 12 atomes de carbone dans lequel 1 ou 2 groupes CH₂ non adjacents peuvent également être remplacés par -O- et/ou -HC=CH-,
Q représente 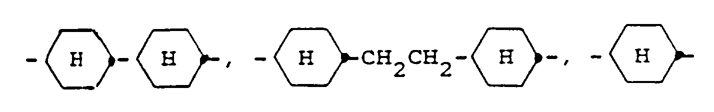 ou une liaison simple,
m vaut 0 ou 1 , et
l'un des radicaux L¹ et L² présents dans la molécule est F et l'autre radical - s'il y en a - est l'hydrogène.

2. Dérivés de benzène de la revendication 1 , caractérisés par la formule Ia 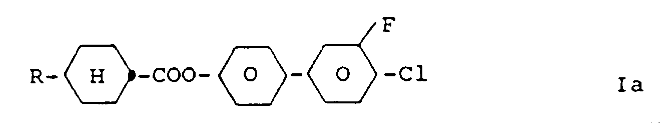 dans laquelle R a la signification donnée dans la revendication I .

3. Dérivés de benzène de la revendication 1 , caractérisés par la formule Ib 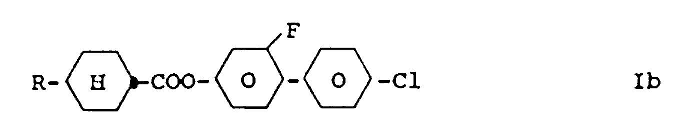 dans laquelle R a la signification donnée dans la revendication 1 .

4. Dérivés de benzène de la revendication 1 , caractérisés par la formule Ic 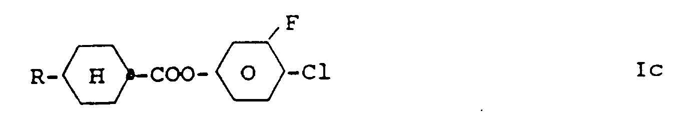 dans laquelle R a la signification donnée dans la revendication I .

5. Dérivés de benzène de la revendication 1 , caractérisés par la formule Id 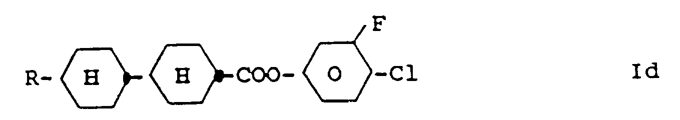 dans laquelle R a la signification donnée dans la revendication 1 .

6. Milieu cristallin liquide qui est un mélange d'au moins deux composés, caractérisé en ce qu'au moi ns un composé est un dérivé de benzène selon la revendication 1 .

7. Dispositif d'affichage à cristaux liquides, caractérisé en ce qu'il contient un milieu cristallin liquide selon la revendication 6 .

8. Dispositif d'affichage électrooptique, caractérisé en ce qu'il contient un milieu cristallin liquide selon la revendication 6 .
